# FASCICULE DE BREVET EUROPEEN

(11) **EP 1 077 657 B1**
(45) Date de publication et mention de la délivrance du brevet: **27.08.2003**
(21) Numéro de dépôt: 99915857.9
(22) Date de dépôt: 28.04.1999
(51) Int. Cl.: A61F 2/16

(54) **DISPOSITIF DE PLIAGE DE LENTILLE INTRAOCULAIRE**
VORRICHTUNG ZUM FALTEN EINER INTRAOKULAREN LINSE
DEVICE FOR BENDING AN INTRAOCULAR LENS

(30) Priorité: 15.05.1998 FR 9806155
(43) Date de publication de la demande: 28.02.2001
(73) Titulaire: Chauvin Opsia, 31320 Castanet-Tolosan (FR)
(72) Inventeur: PYNSON, Joel, F-31400 Toulouse (FR); DAVID, Florian, 31130 Balma (FR)
(74) Mandataire: Cabinet BARRE LAFORGUE & associés
(86) Numéro de dépôt international: FR9901006
(87) Numéro de publication internationale: WO99059504

(56) Documents cités:
- US-A- 5 281 227
- US-A- 5 441 045
- US-A- 5 702 400

## Description

L'invention concerne un dispositif de pliage d'une lentille intraoculaire à partie optique souple avant son implantation à l'état plié par passage au travers d'une incision de faible dimension (classiquement de 3 à 4 mm) ménagée dans l'oeil (en général dans la cornée).

Les lentilles intraoculaires à partie optique souple présentent l'avantage de pouvoir être pliées avant d'être introduites dans l'oeil, ce qui permet de les passer dans une incision de faible dimension. Après la mise en place dans l'oeil, la partie optique se déplie et reprend sa forme initiale.

Les lentilles intraoculaires qui peuvent être pliées ont une partie optique en un matériau souple qui peut être choisi notamment parmi les élastomères de polyuréthane, les élastomères de silicone, les gels synthétiques ou organiques (hydrogel, PMMA et/ou HEMA hydratés...).

Les lentilles intraoculaires comportent en outre une partie haptique de fixation à la paroi interne de l'oeil qui peut être soit formée du même matériau que la partie optique, soit au contraire formée d'anses haptiques en un autre matériau, par exemple en PMMA, rapportées sur la partie optique.

Certaines lentilles intraoculaires à partie optique souple peuvent être pliées, au choix du chirurgien, selon l'une ou l'autre de plusieurs lignes de pliage distinctes, choisie notamment en fonction de la forme de la lentille, de son site ou de son mode d'implantation, ou de l'habitude du chirurgien.

Ainsi, les lentilles intraoculaires comprenant deux anses haptiques courbes dites en "C" peuvent être pliées globalement selon l'un ou l'autre de deux modes de pliage.

Dans le premier mode de pliage, dit 6h-12h, la partie optique est pliée selon une ligne de pliage diamétrale dont les prolongements au-delà de la partie optique coupent la partie d'extrémité libre de chaque anse haptique.

Une fois la lentille pliée, celle-ci présente les deux anses haptiques qui s'étendent longitudinalement à l'opposé l'une de l'autre de la partie optique repliée. L'implantation s'effectue alors en deux étapes. Dans une première étape, le chirurgien tenant une pince entre les mors desquels la partie optique repliée est serrée, introduit la première anse haptique puis la partie optique dans l'oeil (dans le sac capsulaire pour une opération de la cataracte), puis desserre la pince pour libérer la partie optique. Dans une deuxième étape, le chirurgien serre dans la pince la deuxième anse haptique qui s'étend encore à travers l'incision, et l'introduit dans l'oeil.

Ce premier mode de pliage présente l'avantage d'être relativement facile à exécuter. Mais il nécessite deux étapes d'implantation et deux passages de la pince à travers l'incision. L'opération est donc relativement longue, et ces passages répétés à travers l'incision sont un facteur de risques pour le patient.

Dans le deuxième mode de pliage, dit 3h-9h, la partie optique est pliée selon une ligne de pliage diamétrale dont les prolongements au-delà de la partie optique ne coupent pas les anses haptiques. Une fois la lentille pliée, celle-ci présente les deux anses haptiques qui se chevauchent et se croisent, du même côté de la partie optique repliée. L'implantation peut alors s'effectuer en une seule étape, en introduisant tout d'abord les extrémités libres des deux anses haptiques, puis, après une rotation, la partie optique, à travers l'incision.

Avec ce deuxième mode de pliage, l'opération d'implantation est plus rapide, mais beaucoup plus délicate, car le chirurgien doit contrôler le bon passage et le positionnement correct de la lentille dans l'incision, et lors du dépliage de la partie optique, dans l'oeil. En outre, il peut arriver que les anses haptiques, présentées dans l'incision avec leur extrémité libre vers l'avant, butent sur une paroi oculaire, ce qui peut entraîner des lésions de l'oeil, voire même une cassure d'une anse haptique.

Dès lors, aucun mode de pliage ne l'emporte sur l'autre, et les inventeurs ont en fait constaté que, pour une même lentille intraoculaire, il existe aussi bien des chirurgiens qui préconisent et utilisent le premier mode de pliage 6h-12h, et d'autres chirurgiens qui préconisent et utilisent le deuxième mode de pliage 3h-9h. En outre, il a été constaté que le mode de pliage retenu peut varier selon la personnalité du chirurgien, c'est-à-dire selon son goût ou ses habitudes, mais aussi, pour un même chirurgien, selon les cas d'opérations chirurgicales à traiter, de sorte qu'il n'est pas possible de savoir, à l'avance, le mode de pliage que le chirurgien retiendra pour une opération.

US-5 290 293 décrit une pince de pliage en une seule pièce comprenant des logements de réception de l'implant, et une paire de portées de pliage qui se rapprochent sensiblement en translation selon une direction diamétrale de la lentille lorsqu'on actionne des poignées de manoeuvre reliées entre elles par une portion souple formant charnière. Avec ce dispositif, la lentille intraoculaire une fois placée dans les logements de réception ne peut être pliée que selon une seule ligne de pliage perpendiculaire à ladite direction diamétrale, uniquement selon le mode de pliage 6h-12h. En outre, la lentille doit tout d'abord être manipulée pour être mise en place dans les logements de réception de la pince en vue de son pliage.

US-5 139 501 décrit un dispositif de pliage comprenant une portée de pliage fixe sur un bâti, et une portée de pliage formée à l'extrémité d'un coulisseau monté mobile en translation par rapport au bâti, avec des plots d'attache des anses haptiques. Ce dispositif permet un pliage uniquement selon le mode de pliage 3h-9h. En outre, il est à noter que le coulisseau est guidé en translation vers la lentille dans une glissière en queue d'aronde, de sorte que les éventuelles poussières d'usure résultant du frottement des pièces lors de ce déplacement tendent à être emportées vers la lentille, avec le risque de la salir avant l'implantation.

Ces dispositifs de pliage ne connaissent qu'un faible succès commercial. En effet, lorsqu'ils sont proposés conjointement avec la lentille (par exemple comme emballage de la lentille placée en usine dans le dispositif), le chirurgien ne peut utiliser la lentille qu'avec le mode de pliage du dispositif de pliage correspondant. Il est donc nécessaire soit de prévoir, avant l'achat, le mode pliage retenu, ce qui est rarement possible, soit de constituer des stocks avec chaque type de dispositif de pliage. Par ailleurs, si le dispositif de pliage est proposé indépendamment de la lentille, des manipulations à risque sont nécessaires pour placer la lentille dans le dispositif de pliage, ce qui augmente le temps nécessaire à l'opération et les risques de salissure et de détérioration de la lentille.

US-5.281.227 décrit un dispositif de pliage de lentille souple comprenant un bâti, un organe plieur monodirectionnel comprenant une paire de mâchoires de pliage portées par des longerons souples élastiques de cet organe plieur monté rotatif sur le bâti et dissociable du bâti, et un capot. Pour plier la lentille en place entre les mâchoires de l'organe plieur, on ôte le capot, puis on place l'organe plieur selon l'orientation souhaitée par pivotement par rapport au bâti, le bâti empêchant la lentille de pivoter sur son support, puis on dissocie l'organe plieur du bâti, la lentille restant en place entre les mâchoires de l'organe plieur, puis on insère une pince de préhension de la lentille entre les mâchoires de l'organe plieur, puis on rapproche les deux mâchoires l'une vers l'autre par déformation élastique des longerons. Ce dispositif présente notamment l'inconvénient de nécessiter un glissement du support de lentilles de l'organe plieur par rapport à la lentille lors du pivotement d'orientation de l'organe plieur, au risque de détériorer l'état de surface de la partie optique, particulièrement sensible, de la lentille. De surcroît, le pliage de la lentille requiert un grand nombre de manipulations dont certaines sont relativement délicates, de la part du chirurgien. En outre, en cas de difficulté lors du pliage, la lentille risque de chuter à terre et être définitivement perdue. Egalement, le chirurgien ne peut pas laisser l'organe plieur dans une position intermédiaire de pliage compte tenu de l'élasticité des longerons. Et, l'action du chirurgien sur les longerons se retrouve directement, sans aucune démultiplication, sur les mâchoires de pliage et sur la lentille de sorte que le pliage ne peut pas être réalisé progressivement et précisément. La qualité du pliage est donc étroitement liée à l'habileté du chirurgien.

L'invention vise donc à pallier ces inconvénients en proposant un dispositif de pliage d'une lentille intraoculaire qui permette, sans manipulation préalable de la lentille par rapport au dispositif, de plier la lentille selon l'un ou l'autre de plusieurs modes de pliage correspondant à des lignes de pliage diamétrales distinctes, et ce au choix du chirurgien immédiatement avant l'opération d'implantation.

L'invention vise ainsi en particulier à proposer un dispositif de pliage qui permette de réaliser le pliage d'une lentille intraoculaire à anses haptiques en C, au choix du chirurgien immédiatement avant l'opération d'implantation, sans manipulation préalable de la lentille par rapport au dispositif, soit selon un mode de pliage 6h-12h, soit selon un mode de pliage 3h-9h.

L'invention vise également à proposer un tel dispositif de pliage qui soit simple et peu coûteux.

L'invention vise plus particulièrement à proposer des modes de réalisation d'un tel dispositif de pliage dans lesquels il est constitué d'un nombre minimum de pièces distinctes, notamment d'au plus quatre pièces mobiles l'une par rapport à l'autre.

L'invention vise aussi, et plus particulièrement, à proposer des modes de réalisation d'un tel dispositif de pliage qui présentent des risques limités de salissure et de détérioration de la lentille au cours du pliage.

L'invention vise aussi, et plus particulièrement, à proposer des modes de réalisation d'un tel dispositif de pliage qui soient d'usage simple et immédiatement compréhensible, notamment qui permettent de choisir le mode de pliage facilement, rapidement, ct sans risque d'erreur.

L'invention vise aussi, et plus particulièrement, à proposer des mode de réalisation d'un tel dispositif de pliage dont la manoeuvre soit aisée et rapide, et avec lequel le pliage peut être exécuté progressivement, lentement et avec précision sans nécessiter une habileté particulière de la part du chirurgien. A ce titre, l'invention vise plus particulièrement à proposer des modes de réalisation d'un tel dispositif de pliage dans lesquels le mouvement imparti par le chirurgien sur chaque organe mobile de manoeuvre est démultiplié, la course de déplacement de la portée de pliage mobile correspondante étant réduite par rapport à celle de l'organe mobile de manoeuvre.

L'invention vise aussi, et plus particulièrement, à proposer des modes de réalisation d'un tel dispositif de pliage dans lesquels il puisse servir d'emballage de la lentille intraoculaire et être stérilisé.

Pour ce faire, l'invention concerne un dispositif de pliage d'une lentille intraoculaire à partie optique souple avant son implantation, comprenant:
. des moyens de réception de la lentille,
. au moins une paire de portées de pliage adaptées pour pouvoir être placées au contact de la partie optique de la lentille en deux zones de contact opposées selon une même direction diamétrale,
. des moyens de manoeuvre reliés à au moins une portée de pliage et adaptés, lorsqu'ils sont actionnés, pour en commander un déplacement vers une autre portée de pliage d'une même paire de portées de pliage,
caractérisé en ce que les moyens de manoeuvre sont adaptés pour que, une lentille étant en place dans les moyens de réception:
- selon un premier mode d'actionnement des moyens de manoeuvre, deux portées de pliage viennent au contact de la partie optique de la lentille en deux zones de contact opposées selon une première direction diamétrale, pour un pliage selon une première ligne de pliage,
- selon au moins un deuxième mode d'actionnement des moyens de manoeuvre, deux portées de pliage viennent au contact de la partie optique de la lentille en deux zones de contact opposées selon une deuxième direction diamétrale distincte de la première direction diamétrale, pour un pliage selon une deuxième ligne de pliage distincte de la première ligne de pliage,
de sorte que la lentille peut être pliée selon l'une ou l'autre des différentes lignes de pliage.

Avantageusement et selon l'invention, notamment lorsque la lentille intraoculaire est du type comprenant deux anses haptiques en C, (dans tout le texte, on désigne par cette expression, des anses haptiques courbes déformables en flexion reliées à la périphérie de la partie optique par l'une de leurs extrémités et ayant une autre extrémité libre, les deux anses étant globalement de formes symétriques l'une de l'autre par rapport à l'axe optique de la partie optique), la première direction diamétrale et la deuxième direction diamétrale forment entre elles un angle compris entre 60° et 120°, de sorte que la lentille peut être pliée selon l'une ou l'autre de deux lignes de pliage formant entre elles un angle compris entre 60° et 120°.

Dans une variante, avantageusement et selon l'invention, le dispositif de pliage comprend :
- un bâti comportant les moyens de réception de la lentille,
- au moins une paire de portées de pliage adaptées pour pouvoir être placées radialement au contact de la périphérie de la partie optique de la lentille en deux zones de contact diamétralement opposées, l'une au moins des deux portées de pliage, dite portée de pliage mobile d'une paire de portées de pliage, étant montée par rapport au bâti de façon à pouvoir être rapprochée de l'autre portée de pliage de cette paire de portées de pliage,
et en ce que les moyens de manoeuvre comprennent au moins un organe mobile de manoeuvre relié à au moins une portée de pliage mobile pour en commander les déplacements par rapport aux moyens de réception lorsque cet organe mobile de manoeuvre est actionné.

Selon une caractéristique avantageuse de l'invention, le dispositif de pliage est caractérisé en ce qu'il comprend:
- une première paire de portées de pliage adaptées pour pouvoir être placées au contact de la périphérie de la partie optique de la lentille en deux zones de contact opposées selon la première direction diamétrale, et produire sous l'effet de l'actionnement d'au moins un organe mobile de manoeuvre selon le premier mode d'actionnement, un pliage de la lentille selon la première ligne de pliage,
- au moins une deuxième paire de portées de pliage, distincte de la première paire de portées de pliage, les portées de pliage de cette deuxième paire étant adaptées pour pouvoir être placées au contact de la périphérie de la partie optique de la lentille en deux zones de contact opposées selon la deuxième direction diamétrale et produire, sous l'effet de l'actionnement d'au moins un organe mobile de manoeuvre selon le deuxième mode d'actionnement, un pliage de la lentille selon la deuxième ligne de pliage distincte de la première ligne de pliage.

Dans un mode de réalisation et selon l'invention, le dispositif de pliage est caractérisé en ce que les moyens de manoeuvre comprennent au moins deux organes mobiles de manoeuvre, dont l'un est relié à au moins une première portée de pliage mobile d'une première paire de portées de pliage, et dont l'autre est relié à au moins une deuxième portée de pliage mobile d'une deuxième paire de portées de pliage.

Dans un autre mode de réalisation, avantageusement et selon l'invention, le dispositif de pliage est caractérisé en ce que les moyens de manoeuvre comprennent un organe mobile de manoeuvre relié à au moins une première portée de pliage mobile de la première paire de portées de pliage et à au moins une deuxième portée de pliage mobile de la deuxième paire de portées de pliage, cet organe mobile de manoeuvre étant adapté pour pouvoir être actionné soit selon le premier mode d'actionnement, soit selon le deuxième mode d'actionnement.

Avantageusement et selon l'invention, le dispositif de pliage est caractérisé en ce que l'organe mobile de manoeuvre est monté rotatif par rapport au bâti, en ce que le premier mode d'actionnement correspond à un déplacement en rotation de l'organe mobile de manoeuvre dans un premier sens de rotation, et en ce que le deuxième mode d'actionnement correspond à un déplacement en rotation de l'organe mobile de manoeuvre dans le deuxième sens de rotation opposé au premier sens de rotation. En variante ou en combinaison, le dispositif de pliage est caractérisé en ce que l'organe mobile de manoeuvre est monté par rapport au bâti de façon à pouvoir être déplacé selon au moins deux directions de translation distinctes dont l'une correspond au premier mode d'actionnement alors que l'autre correspond au deuxième mode d'actionnement. Dans une autre variante, ou en combinaison, le dispositif de pliage est caractérisé en ce que l'organe mobile de manoeuvre est monté par rapport au bâti de façon à pouvoir être déplacé selon au moins une direction de translation et selon au moins deux sens opposés selon cette direction de translation, dont l'un correspond au premier mode d'actionnement alors que l'autre correspond au deuxième mode d'actionnement.

Il est à noter que rien n'empêche de prévoir que l'organe mobile de manoeuvre soit monté rotatif et coulissant, c'est-à-dire avec des composantes de déplacement combinées en rotation et en translation.

Par ailleurs, avantageusement et selon l'invention, le dispositif de pliage est caractérisé en ce qu'une première portée de pliage mobile de la première paire de portées de pliage et une deuxième portée de pliage mobile de la deuxième paire de portées de pliage sont solidaires d'une seule et même pièce mobile intermédiaire montée et guidée par rapport au bâti et reliée à l'organe mobile de manoeuvre de façon à pouvoir être entraînée en déplacement sous l'effet de l'actionnement de cet organe mobile de manoeuvre selon l'un ou l'autre des modes d'actionnement

Egalement, dans les différents modes de réalisation, avantageusement et selon l'invention, un organe mobile de manoeuvre est relié à chaque portée de pliage mobile dont il commande les déplacements par l'intermédiaire d'un système à came et coulisseau de contact. Ainsi, le mouvement d'actionnement du praticien sur cet organe de manoeuvre mobile pourra avoir une course plus grande que celle du déplacement de la portée de pliage mobile.

En outre, dans certains modes de réalisation, avantageusement et selon l'invention, chaque paire de portées de pliage comprend une portée de pliage fixe par rapport au bâti. Egalement, dans certains modes de réalisation, avantageusement et selon l'invention, chaque portée de pliage mobile est montée par rapport au bâti, de façon à pouvoir être mobile au moins sensiblement en translation, notamment selon une direction diamétrale correspondante.

En outre, avantageusement et selon l'invention, le dispositif de pliage est caractérisé en ce que les moyens de réception sont adaptés pour recevoir la lentille intraoculaire orientée, notamment par rapport au bâti, selon une et une seule direction possible. En particulier, les moyens de réception comportent, avantageusement et selon l'invention, des logements de réception de la partie haptique de la lentille -notamment des anses haptiques en C-. Les moyens de réception peuvent être formés, en totalité ou en partie, par les portées de pliage elles-mêmes (fixe(s) ou mobile(s)).

Il est à noter que la lentille étant en place dans les moyens de réception selon son orientation prédéterminée, le chirurgien n'a pas à manipuler la lentille, avec une pince ou autrement, pour choisir le mode de pliage. Il lui suffit en effet d'actionner les moyens de manoeuvre du dispositif de pliage selon le mode d'actionnement approprié au pliage retenu.

Par ailleurs, avantageusement et selon l'invention, le dispositif de pliage est caractérisé en ce qu'il constitue un emballage d'une lentille intraoculaire et comprend un capot refermé sur la lentille en place dans les moyens de réception, ce capot étant adapté pour pouvoir être ouvert pour permettre l'accès à la lentille. Avantageusement et selon l'invention, ce capot est adapté pour avoir aussi pour fonction de maintenir la lentille en place dans les moyens de réception perpendiculairement au plan de la partie optique, c'est-à-dire selon l'axe optique.

En outre, avantageusement et selon l'invention, le capot est adapté pour, en position refermée sur la lentille, interdire tout déplacement intempestif des moyens de manoeuvre, et, en position ouverte, autoriser l'actionnement des moyens de manoeuvre selon l'un ou l'autre des modes d'actionnement.

L'invention s'étend aussi à un dispositif de pliage caractérisé en combinaison par tout ou partie des caractéristiques mentionnées ci-dessus ou ci-après.

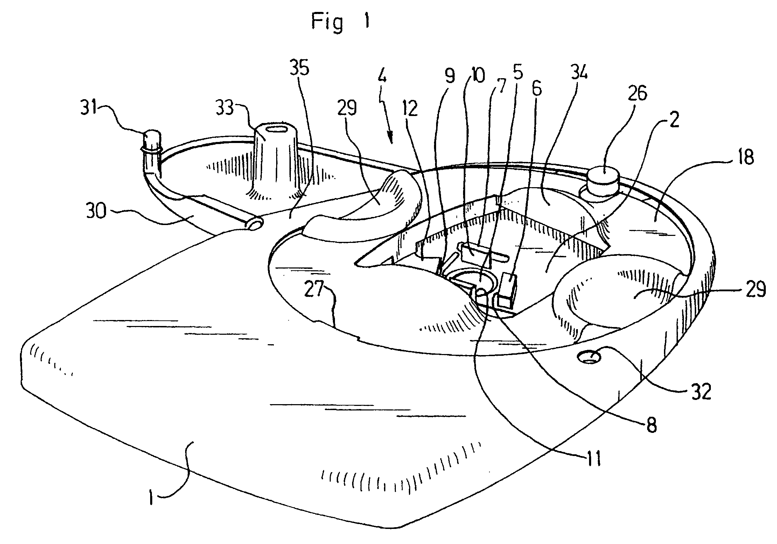

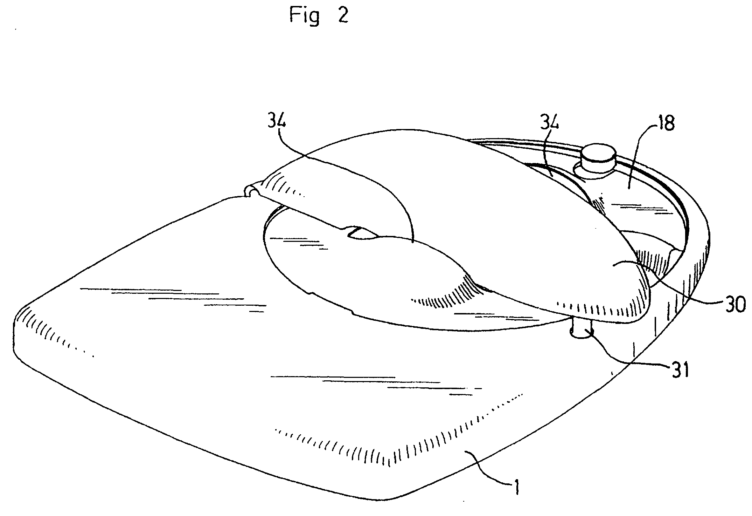

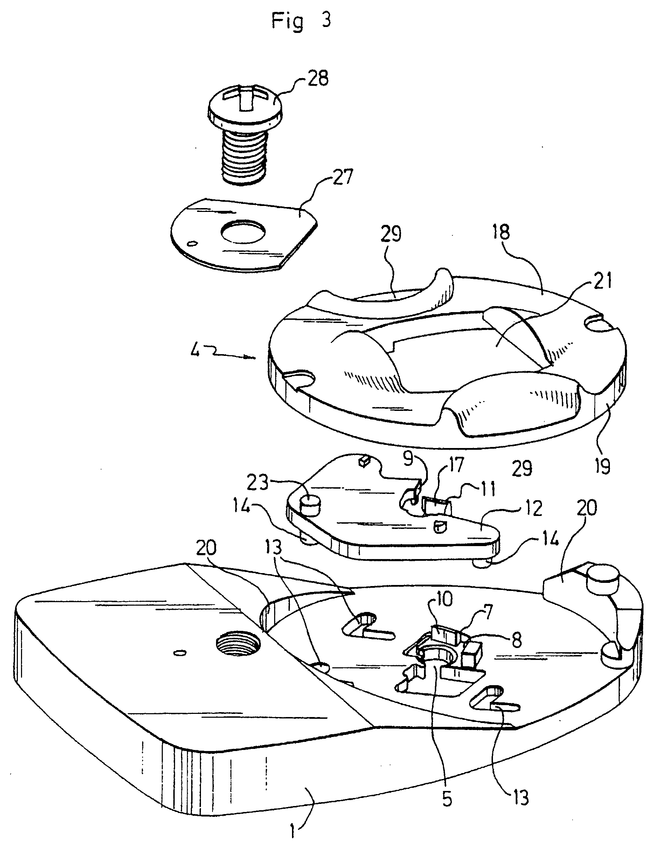

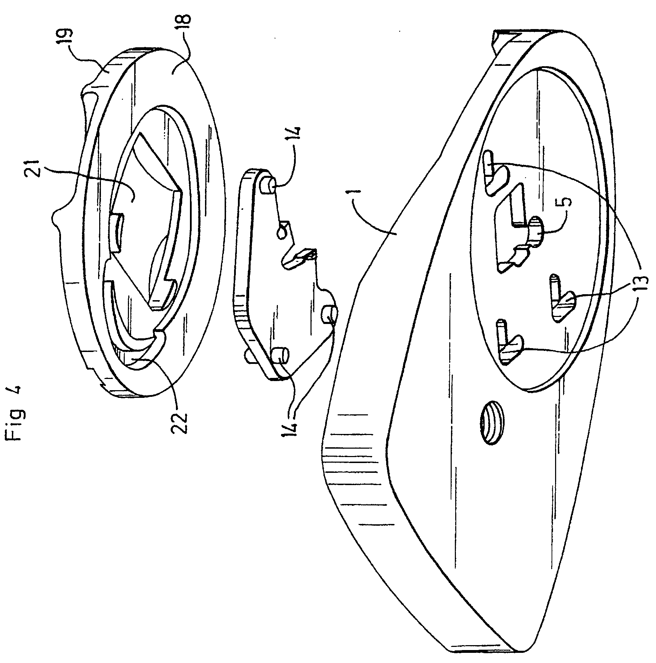

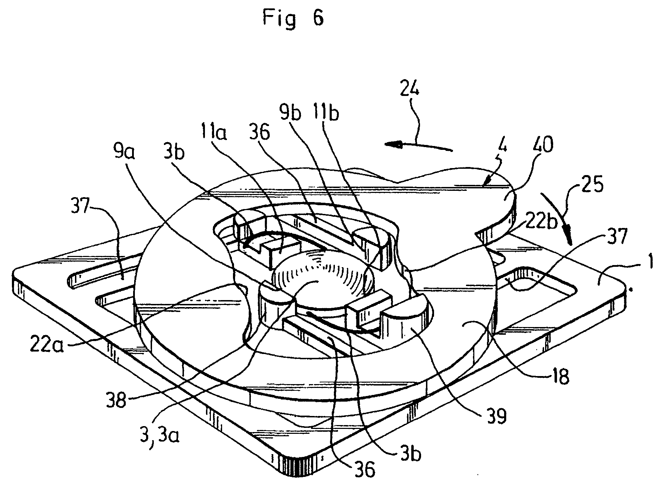

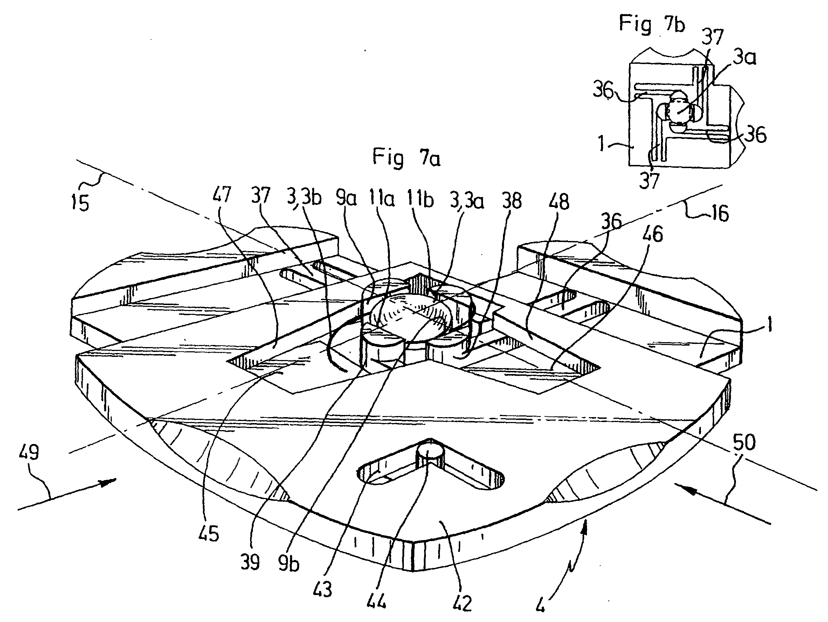

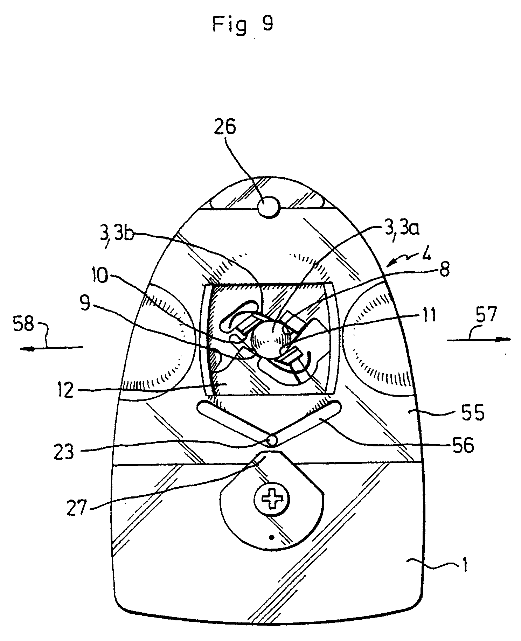

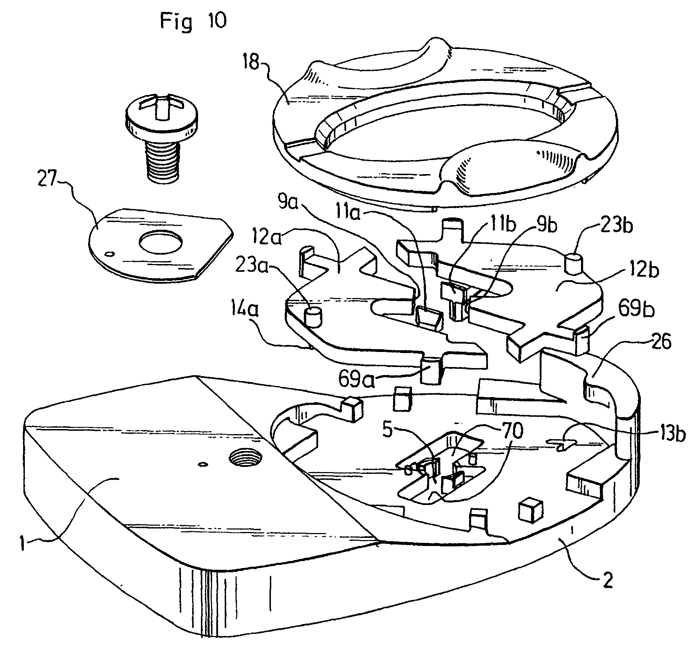

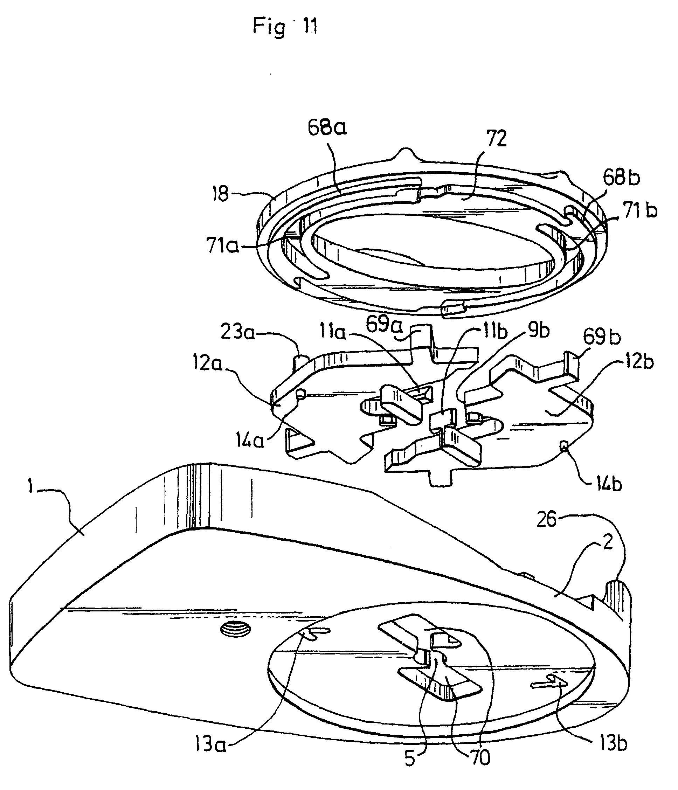

D'autres buts, caractéristiques et avantages de l'invention apparaîtront à la lecture de la description suivante qui se réfère aux figures annexées dans lesquelles :
. les figures 1 et 2 sont des vues schématiques en perspective d'un dispositif de pliage selon un premier mode de réalisation de l'invention réalisé en matière synthétique, illustré, respectivement, avec le capot ouvert et avec le capot fermé.
. les figures 3 et 4 sont des vues schématiques en perspective éclatée de trois-quarts supérieure et, respectivement de trois-quarts inférieure, d'un dispositif de pliage selon un deuxième mode de réalisation semblable au premier mode de réalisation de l'invention, mais réalisé en métal et exempt de capot,
. les figures Sa, 5b, 5c sont des vues schématiques de dessus illustrant le dispositif de pliage des figures 3 et 4, respectivement en position initiale, en position de pliage 6h-12h, et en position de pliage 3h-9h d'une lentille intraoculaire,
. les figures 6, 7a et 8 sont des vues schématiques en perspective illustrant, respectivement, trois autres modes de réalisation d'un dispositif de pliage selon l'invention, la figure 7b étant une vue schématique de dessus, à moindre échelle, du bâti du dispositif de la figure 7a,
. la figure 9 est une vue schématique de dessus illustrant un autre mode de réalisation d'un dispositif selon l'invention,
. les figures 10 et 11 sont des vues schématiques en perspective éclatée de trois-quart supérieure et, respectivement de trois-quart inférieure d'un autre mode de réalisation d'un dispositif selon l'invention.

Sur les différentes figures, les mêmes repères sont utilisés pour désigner les mêmes éléments fonctionnels.

Le premier mode de réalisation des figures 1 et 2, et le deuxième mode de réalisation des figures 3 et 4, présentent les mêmes éléments fonctionnels et la même cinématique, du point de vue du pliage de la lentille intraoculaire. Ils ne diffèrent que par le matériau utilisé (matière synthétique dans les figures 1 et 2, métal tel que l'aluminium dans les figures 3 et 4) et par le fait que le premier mode de réalisation est doté d'un capot 30 articulé, et peut faire office d'emballage pour la lentille 3 intraoculaire.

Sur ces figures, le dispositif de pliage comporte un bâti 1 définissant un fond 2 de réception d'une lentille intraoculaire 3 à plier en vue de son implantation dans l'oeil d'un patient. Le bâti 1 est globalement plat et présente des formes et dimensions qui sont de préférence adaptées pour permettre la préhension du dispositif dans une main, l'autre main servant à actionner des moyens 4 de manoeuvre montés sur le bâti 1 pour le pliage de la lentille intraoculaire 3. Dans la présente description, le bâti 1 et le fond 2 sont supposés s'étendre horizontalement. La lentille intraoculaire 3 comprend une partie optique 3a en matériau souple pliable et deux anses haptiques 3b en C.

Le fond 2 de réception est avantageusement doté d'un évidement ou perçage central cylindrique 5, d'axe vertical, en regard de la partie optique 3a, de diamètre inférieur à celui de la partie optique 3a, pour faciliter le pliage et minimiser les frottements des bords périphériques de la partie optique 3a sur le fond 2 au cours du pliage. Le diamètre de l'évidement ou perçage central 5 est par exemple de l'ordre de 4mm.

Le fond 2 porte deux plots fixes 6, 7, disposés au moins sensiblement à 90° l'un de l'autre autour et à proximité de l'évidement ou perçage central 5. Chacun de ces plots 6, 7 définit une face 8, respectivement 10, qui s'étend vers le haut à partir du fond 2, et au moins sensiblement perpendiculairement à la direction radiale de l'évidement ou perçage central 5 et de la partie optique 3a d'une lentille en place sur le fond 2. Ces faces 8, 10 définissent deux portées de pliage fixes 8, 10 adaptées pour venir au contact de la périphérie de la partie optique 30.

Le dispositif comporte une pièce mobile intermédiaire 12 montée et guidée sur le bâti 1 grâce à au moins une rainure en V 13 du fond 2 recevant au moins un téton 14 de la pièce mobile intermédiaire 12, notamment grâce à un ensemble de rainures 13 et tétons 14. Comme on le voit figures 3 et 4, le fond 2 comporte trois rainures en V 13, ayant chacune deux branches, à savoir une première branche et une deuxième branche formant entre elles un angle au moins sensiblement égal à l'angle formé entre les deux lignes de pliage potentiel souhaitées pour la lentille 3. Les différentes rainures 13 (c'est-à-dire leurs branches correspondantes) sont parallèles entre elles. La pièce mobile intermédiaire 12 comporte trois tétons 14 s'étendant en saillie vers le bas pour pouvoir coulisser dans les trois rainures 13. La pièce mobile intermédiaire 12 peut ainsi se déplacer en translation par rapport au fond 2 selon l'une ou l'autre des directions correspondant aux directions des deux branches des rainures 13.

La pièce mobile intermédiaire 12 présente deux faces 9, respectivement 11, qui s'étendent vers le haut par rapport au fond 2. Lorsque la pièce mobile intermédiaire 12 est en position initiale avant le pliage, les tétons 14 étant placés au sommet des rainures en V 13, ces faces 9, 11 s'étendent au moins sensiblement perpendiculairement à la direction radiale de l'évidement ou perçage central 5 et de la partie optique 3a d'une lentille en place sur le fond 2.

Chacune de ces faces 9, 11 qui se déplace avec la pièce mobile intermédiaire 12 définit une portée de pliage mobile 9, 11 adaptée pour venir au contact de la périphérie de la partie optique 3a de la lentille.

Le dispositif de pliage comporte ainsi deux paires 8, 9 et 10, 11 de portées de pliage.

La première paire 8, 9 comprend une portée de pliage fixe 8 solidaire du fond 2 et une portée de pliage mobile 9 solidaire de la pièce mobile intermédiaire 12, et ces deux portées de pliage 8, 9 sont adaptées pour pouvoir être placées au contact de la périphérie de la partie optique 3a de la lentille 3 en place sur le fond 2 en deux zones de contact opposées selon une première direction diamétrale 15 au moins sensiblement parallèle à la direction des premières branches des rainures en V 13.

La deuxième paire 10, 11 comprend une portée de pliage fixe 10 solidaire du fond 2 et une portée de pliage mobile 11 solidaire de la pièce mobile intermédiaire 12, et ces deux portées de pliage 10, 11 sont adaptées pour pouvoir être placées au contact de la périphérie de la partie optique 3a de la lentille 3 en place sur le fond 2 en deux zones de contact opposées selon une deuxième direction diamétrale 16 au moins sensiblement perpendiculaire à la première direction diamétrale 15, cette deuxième direction diamétrale 16 étant au moins sensiblement parallèle à la direction des deuxièmes branches des rainures en V 13.

En position initiale, les deux portées de pliage mobile 10, 11 sont disposées autour de l'évidement ou perçage central 5, au moins sensiblement à 90° l'une de l'autre autour et à proximité de cet évidement ou perçage central 5. Les deux portées de pliage 8, 9 et 10, 11 d'une même paire s'étendent au moins sensiblement parallèles l'un de l'autre et perpendiculairement à la direction diamétrale 15, 16 correspondante.

La pièce mobile intermédiaire 12 est adaptée pour permettre le passage d'une anse haptique 3b autour et derrière l'une 11 des deux portées de pliage mobile (celle de la deuxième paire), et comporte un évidement 17 pour ce faire. L'autre anse haptique 3b de la lentille 3 peut être passée derrière le plot 7 définissant la portée de pliage fixe 10 de la deuxième paire. La pièce mobile intermédiaire 12 et/ou l'autre plot 6 sont adaptés pour empêcher le passage des anses haptiques 3b derrière les autres portées de pliage 8, 9 de la première paire de portées de pliage. De la sorte, la lentille 3 ne peut être placée que selon une seule orientation possible par rapport au fond 2.

Le dispositif de pliage comporte aussi un organe mobile de manoeuvre 18 rotatif en forme générale de couronne qui fait office de moyen de manoeuvre. Cet organe mobile de manoeuvre 18 est évidé en sa partie centrale pour présenter une lumière d'accès 21 en regard de la lentille 3 en place sur le fond 2, et présente un chant externe circulaire 19 venant glisser contre une ou plusieurs face(s) cylindrique(s) 20 de guidage solidaires du bâti 1 adaptées pour recevoir l'organe mobile de manoeuvre 18 et le guider en rotation par rapport au bâti 1.

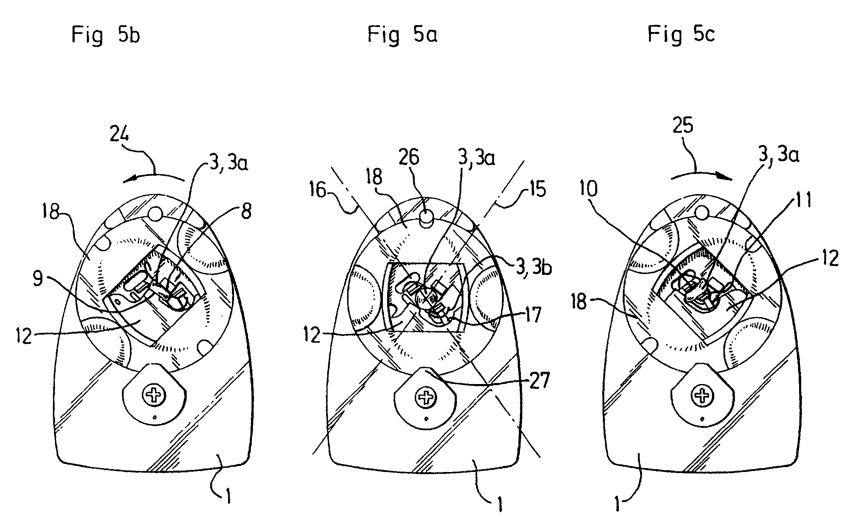

L'organe mobile de manoeuvre rotatif 18 est relié aux portées de pliage mobiles 9, 11, c'est-à-dire à la pièce mobile intermédiaire 12, par l'intermédiaire d'un mécanisme à came 22 et coulisseau de contact 23. Ainsi, la pièce mobile intermédiaire 12 porte un coulisseau 23 qui s'étend en saillie vers le haut pour coopérer avec une came 22 ménagée en creux sous l'organe mobile de manoeuvre 18. La forme de came 22 est adaptée pour que lorsque l'organe mobile de manoeuvre 18 est déplacé dans un premier sens de rotation 24 (figure 5b), la pièce mobile intermédiaire 12 est déplacée selon la première direction diamétrale 15 (les tétons 14 étant guidés dans les premières branches des rainures 13), et lorsqu'il est déplacé dans le deuxième sens de rotation 25 (figure 5c) opposé au premier sens de rotation 24, la pièce mobile intermédiaire 12 est déplacée selon la deuxième direction diamétrale 16 (les tétons 14 étant guidés dans les deuxièmes branches des rainures 13).

L'organe mobile de manoeuvre 18 vient coiffer la pièce mobile intermédiaire 12 et l'ensemble est maintenu en place axialement par rapport au bâti 1 par des butées 26, 27.

Dans le mode de réalisation des figures 3 et 4, le dispositif comprend une butée fixe 26 s'étendant en saillie horizontalement au-dessus de la bordure de la face supérieure de l'organe mobile de manoeuvre 18 et, diamétralement opposée à cette butée fixe 26, une butée amovible 27 fixée par une vis 28, de façon à permettre le montage et le démontage de l'organe mobile de manoeuvre 18.

Dans le mode de réalisation des figures 1 et 2, les deux butées 26, 27 sont fixes, et l'organe mobile de manoeuvre 18 est engagé en force sous ces butées 26, 27 en matière plastique par déformation élastique.

L'organe mobile de manoeuvre 18 comprend deux échancrures de manoeuvre 29 permettant de faciliter sa prise manuelle et son actionnement en rotation.

Le premier mode d'actionnement (figure 5b) de l'organe mobile de manoeuvre 18 selon le premier sens de rotation 24 a pour effet de rapprocher radialement la portée de pliage mobile 9 de la première paire de portées de pliage, de la portée de pliage fixe 8 de cette première paire, selon la première direction radiale 15, ce qui entraîne le pliage de la partie optique 3a de la lentille selon une première ligne de pliage 6h-12h au moins sensiblement perpendiculaire à la première direction diamétrale 15.

Le deuxième mode d'actionnement (figure 5c) de l'organe mobile de manoeuvre 18 selon le deuxième sens de rotation 25 a pour effet de rapprocher radialement la portée de pliage mobile 11 de la deuxième paire de portées de pliage, de la portée de pliage fixe 10 de cette deuxième paire, selon la deuxième direction radiale 16, ce qui entraîne le pliage de la partie optique 3a de la lentille selon une deuxième ligne de pliage 3h-9h au moins sensiblement perpendiculaire à la deuxième direction diamétrale 16.

Le capot 30 du dispositif de pliage du premier mode de réalisation est articulé sur le côté du bâti 1, par exemple par une charnière film 35 formée d'une bande mince de matériau synthétique. Ce capot 30 comporte un téton de verrouillage 31 en position fermée engagé en force dans un orifice 32 du bâti 1, et une extension axiale 33 s'étendant vers le bas en position fermée pour maintenir une lentille 3 axialement en place sur le fond 2 de réception. En outre, en position fermée, le capot 30 est engagé entre deux parois droites 34 de l'organe mobile de manoeuvre 18, de sorte qu'il interdit alors tout déplacement intempestif en rotation de cet organe mobile de manoeuvre 18.

Lorsque le capot est ouvert (figure 1), l'organe mobile de manoeuvre 18 est libéré et peut être actionné dans l'un ou l'autre des sens de rotation 24, 25.

Dans les modes de réalisation des figures 1 et 2 et 3 et 4, le dispositif de pliage selon l'invention est essentiellement constitué de trois pièces mobiles les unes par rapport aux autres, à savoir le bâti 1, la pièce mobile intermédiaire 12 et l'organe mobile de manoeuvre 18.

Dans le mode de réalisation représenté figure 6, le dispositif de pliage n'est formé que de deux pièces : le bâti 1 et l'organe mobile de manoeuvre 18 monté rotatif sur ce bâti 1. Chaque portée de pliage mobile 9a, 9b, 1 la, 11b est formée par un plot d'extrémité d'un bras flexible 36, 37 solidaire du bâti 1 s'étendant horizontalement et perpendiculairement à la direction diamétrale selon laquelle la portée doit se déplacer.

En outre, dans ce mode de réalisation, toutes les portées de pliage sont mobiles. La première paire de portées de pliage comprend deux portées de pliage mobiles 9a, 9b et la deuxième paire de portées de pliage comprend deux portées de pliage mobiles 11a, 11b. L'organe mobile de manoeuvre 18 comporte deux cames 22a, 22b délimitant sa lumière centrale 21, et adaptées pour venir simultanément au contact des faces postérieures semi-cylindriques 38 ou 39 des deux plots formant les deux portées de pliage 9a, 9b ou 11a, 11b d'une même paire de portées de pliage. L'une 22a des cames commande l'une 9a, 1 la des portées de pliage, tandis que l'autre came 22b commande l'autre portée de pliage 9b, 11b. Les portées de pliage 9a, 9b de la première paire présentent des échancrures formant des logements de réception des anses haptiques 3b de la lentille. Dans la variante représentée la lentille 3 est maintenue en place axialement, c'est-à-dire portée par le bâti 1, uniquement grâce à ces logements de réception, par ses anses haptiques 3b.

Les portées de pliage mobiles 9a, 9b, 11a, 11b peuvent présenter, comme pour le mode de réalisation de la figure 7b représenté, des extensions radiales vers l'intérieur sous la partie optique 3a de la lentille de façon à former, au moins en partie, des moyens de réception de la lentille 3 et la porter axialement.

L'organe mobile de manoeuvre 18 comporte une patte 40 d'actionnement et est guidé sur le bâti 1 par des moyens de guidage en rotation à rainure(s) circulaire(s) et téton(s). Lorsque l'on actionne l'organe mobile de manoeuvre 18 dans le premier sens de rotation 24, les cames 22a, 22b viennent au contact des faces postérieures 38 des plots formant les deux portées de pliage mobiles 9a, 9b de la première paire qui se rapprochent radialement l'une de l'autre par flexion des bras 36 qui les portent. Ce déplacement en flexion est assimilable à une translation selon la première direction diamétrale 15 compte tenu de la longueur des bras 36 et de la faible course de déplacement induite. La lentille 3 est alors pliée selon une ligne de pliage 6h-12h

Lorsque l'on actionne l'organe mobile de manoeuvre 18 dans le deuxième sens de rotation 25, les cames 22a, 22b viennent au contact des faces 39, et les deux portées de pliage mobiles 11a, 11b se rapprochent radialement l'une de l'autre par flexion des bras 37 qui les portent, le déplacement étant assimilable à une translation selon la deuxième direction diamétrale 16. La lentille 3 est pliée selon une ligne de pliage 3h-9h.

Dans le mode de réalisation des figures 7a et 7b, le dispositif de pliage comporte un organe mobile de manoeuvre 42 guidé non pas en rotation sur le bâti 1, mais en translation selon l'une ou l'autre des deux directions 49, 50 perpendiculaires à la première direction diamétrale 15 et, respectivement, à la deuxième direction diamétrale 16. Pour ce faire, au moins une rainure en V 43, à deux branches à 90°, est ménagée dans l'organe mobile de manoeuvre 42, et un téton 44 solidaire du bâti 1 coulisse dans la rainure en V 43.

L'organe mobile de manoeuvre 42 comprend deux lumières 45, 46 trapézoïdales, à 90° l'une de l'autre, définissant chacune une paire de cames droites 47, 48 convergentes l'une par rapport à l'autre pour les faces postérieures 38, 39 des plots portant les portées de pliage mobiles 9a, 9b, 11a, 11b.

Là encore, le bâti 1 porte deux paires de portées de pliage 9a, 9b, 11a, 11b mobiles formées par des plots à l'extrémité de bras flexibles 36, 37 du bâti 1. Chaque plot comporte aussi une extension radiale (figure 7b) vers l'intérieur sous la partie optique 3a de la lentille pour la recevoir et la porter axialement. La longueur radiale de ces extensions n'est pas trop importante pour ne pas empêcher un pliage correct de la lentille 3.

Lorsque l'on actionne l'organe 42 selon une première direction de translation 49 par rapport au bâti 1, les deux cames 47 de la première lumière 45 viennent au contact des faces postérieures 38 des plots portant les deux portées de pliage mobiles 9a, 9b de la première paire, et les rapprochent radialement l'une de l'autre selon la première direction diamétrale 15. La lentille 3 est pliée selon une ligne de pliage 6h-12h.

Lorsqu'on déplace l'organe 42 selon la deuxième direction de translation 50, les deux cames 48 de la deuxième lumière 46 viennent au contact des faces postérieures 39 des plots portant les portées de pliage mobiles 11a, 11b de la deuxième paire, et les rapprochent radialement l'une de l'autre selon la deuxième direction diamétrale 16. La lentille 3 est pliée selon une ligne de pliage 3h-9h.

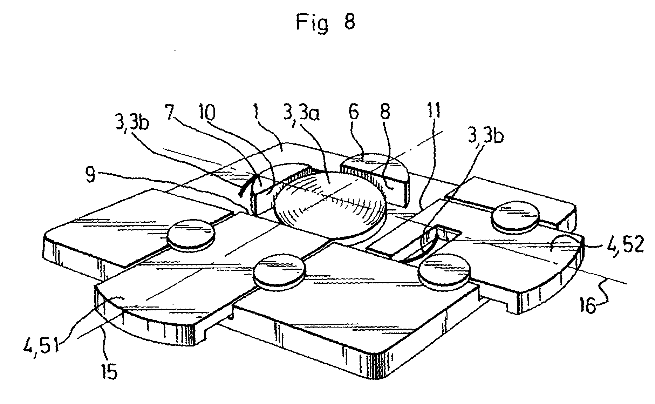

Le mode de réalisation de la figure 8 diffère du précédent par le fait que l'organe mobile de manoeuvre 42 est remplacé par deux organes mobiles de manoeuvre 51, 52, guidés en translation sur le bâti 1, un pour chaque paire de portées de pliage. En outre, dans ce mode de réalisation, le dispositif comprend une première paire de portées de pliage 8, 9 constituée d'une portée de pliage fixe 8 solidaire du bâti 1 et d'une portée de pliage mobile 9 qui est formée de la face d'extrémité du premier organe mobile de manoeuvre 51. De même, la deuxième paire est constituée d'une portée de pliage fixe 10 solidaire du bâti 1 et d'une portée de pliage mobile 11 qui est formée de la face d'extrémité du deuxième organe mobile de manoeuvre 52.

Chaque organe mobile de manoeuvre 51, 52 est guidé en translation par rapport au bâti 1 selon une direction qui correspond à la direction diamétrale 15, 16 selon laquelle la portée de pliage mobile 9, 11 qu'il porte doit être déplacée. Dans ce mode de réalisation, les directions diamétrales 15, 16, et donc les lignes de pliage correspondantes, sont à 90° l'une de l'autre.

Le mode de réalisation de la figure 9 diffère de celui des figures 3 et 4 par le fait que l'organe mobile de manoeuvre 18 rotatif est remplacé par un organe mobile de manoeuvre 55 guidé en translation selon une seule direction (c'est-à-dire selon un seul axe de translation), dans un sens ou dans l'autre, par rapport au bâti 1.

Le coulisseau 23 de la pièce mobile intermédiaire 12 est engagé dans une came en V 56 de l'organe mobile de manoeuvre 55, les branches de cette came 56 étant adaptées pour que le premier sens de translation 57 de l'organe 55 corresponde au premier mode d'actionnement, c'est-à-dire au déplacement de la portée de pliage mobile 9 de la première paire pour un pliage 6h-12h de la lentille 3, et pour que le deuxième sens de translation 58, opposé au premier, corresponde au deuxième mode d'actionnement, c'est-à-dire au déplacement de la portée de pliage mobile 11 de la deuxième paire pour un pliage 3h-9h de la lentille.

Le mode de réalisation des figures 10 et 11 est semblable à celui des figures 3 et 4, mais comprend deux pièces mobiles intermédiaires 12a, 12b, définissant deux paires de portées de pliage mobiles 9a, 9b, 11a, 11b. chaque pièce mobile intermédiaire 12a, 12b est guidée en translation sur le bâti 1 grâce à au moins une rainure en V 13a, 13b du fond 2 du bâti 1 recevant au moins un téton 14a, 14b solidaire, respectivement, de la pièce mobile intermédiaire 12a, 12b correspondante. Dans le mode de réalisation représenté, une seule rainure 13a, 13b en V est prévue pour chaque pièce mobile 12a, 12b qui est par ailleurs guidée par des portées de guidage circulaire 68a, 68b de l'organe mobile de manoeuvre 18 recevant des ergots 69a, 69b de la pièce 12a, 12b. L'organe mobile de manoeuvre 18 est par ailleurs semblable à celui du mode de réalisation des figures 3 et 4, est en forme de couronne, et est guidé en rotation par rapport au bâti 1. Mais, cet organe mobile de manoeuvre 18 comprend deux cames 71a, 71b ménagées en creux dans sa face inférieure 72 pour recevoir, respectivement, un coulisseau de contact 23a, 23b qui s'étend en saillie vers le haut de chaque pièce mobile intermédiaire 12a, 12b.

Chaque paire de portées de pliage mobiles 9a, 9b et 11a, 11b comprend une première portée 9a, 11a solidaire de la première pièce mobile intermédiaire 12a, et une deuxième portée 9b, 11b solidaire de la deuxième pièce mobile intermédiaire 12b.

La forme des cames 71a, 71b de l'organe mobile de manoeuvre 18 et des moyens 13a, 14a, 13b, 14b de guidage des pièces mobiles intermédiaires 12a, 12b par rapport au bâti 1 sont adaptées pour que lorsque l'organe mobile de manoeuvre 18 est déplacé dans un premier sens de rotation, les deux pièces mobiles intermédiaires 12a, 12b sont déplacées en translation en se rapprochant l'une de l'autre selon une même première direction diamétrale, les portées de pliage mobiles 9a, 9b d'une première paire se rapprochant l'une de l'autre, les tétons 14a, 14b étant guidés dans les premières branches, parallèles l'une de l'autre, des rainures 13a, 13b, et, lorsque l'organe mobile de manoeuvre 18 est déplacé dans le deuxième sens de rotation opposé au premier, les pièces mobiles intermédiaires 12a, 12b sont déplacées en translation en se rapprochant l'une de l'autre selon une même deuxième direction diamétrale distincte de la première, les portées de pliage mobiles 11a, 11b d'une deuxième paire se rapprochant l'une de l'autre, les tétons 14a, 14b étant guidés dans les deuxièmes branches, parallèles l'une de l'autre, des rainures 13a, 13b.

L'organe mobile de manoeuvre 18 vient coiffer les deux pièces mobiles intermédiaires 12a, 12b et est maintenu en place axialement par rapport au bâti 1 grâce aux butées 26, 27.

Par ailleurs, dans ce mode de réalisation, le fond 2 comprend un évidement 5 central de réception de la lentille à plier, et des logements de réception 70 des anses haptiques.

Le mode de réalisation des figures 10 et 11 est donc semblable à celui des figures 3 et 4, et ne s'en différencie essentiellement que par le fait que des portées de pliage fixes 8, 10 des figures 3 et 4 sont remplacées par des portées de pliage mobiles 9b, 11b solidaires d'une deuxième pièce mobile intermédiaire 12b.

Dans le mode de réalisation des figures 10 et 11, le dispositif est essentiellement constitué de quatre pièces mobiles les unes par rapport aux autres, à savoir le bâti 1, les deux pièces mobiles intermédiaires 12a, 12b, et l'organe mobile de manoeuvre 18.

L'invention peut faire l'objet d'autres variantes de réalisation. En outre, certaines caractéristiques des différentes variantes peuvent être combinées entre elles.

L'invention est aussi applicable pour le pliage de lentilles intraoculaires ayant des parties haptiques qui ne sont pas des anses en C.

Par ailleurs, il est aussi possible de prévoir plus de deux lignes de pliage distinctes pour un même boîtier de pliage selon l'invention.

## Revendications

1. Dispositif de pliage d'une lentille intraoculaire (3) à partie optique (3a) souple avant son implantation, comprenant:
. des moyens (2) de réception de la lentille (3),
. au moins une paire de portées de pliage (8, 9, 9a, 9b, 10, 11, 11a, 11b) adaptées pour pouvoir être placées au contact de la partie optique (3a) de la lentille (3) en deux zones de contact opposées selon une même direction diamétrale,
. des moyens (4) de manoeuvre reliés à au moins une portée de pliage (9, 9a, 9b, 11, 11a, 11b) et adaptés pour, lorsqu'ils sont actionnés, en commander un déplacement vers une autre portée de pliage (8, 9b, 11b, 11a) d'une même paire de portées de pliage,
**caractérisé en ce que** les moyens (4) de manoeuvre sont adaptés pour que, une lentille (3) étant en place dans les moyens (2) de réception:
- selon un premier mode d'actionnement des moyens (4) de manoeuvre, une première paire de portées de pliage (8, 9, 9a, 9b) vient au contact de la périphérie de la partie optique (3a) de la lentille (3) en deux zones de contact opposées selon une première direction diamétrale (15), cette première paire de portées de pliage (8, 9, 9a, 9b) étant adaptée pour produire, sous l'effet de l'actionnement d'au moins un organe (18, 42, 51, 52, 55, 62a, 62b) mobile de manoeuvre selon ce premier mode d'actionnement, un pliage de la lentille selon une première ligne de pliage,
- selon au moins un deuxième mode d'actionnement des moyens (4) de manoeuvre, une deuxième paire de portées de pliage (10, 11, 11a, 11b), distincte de la première paire de portées de pliage vient au contact de la périphérie de la partie optique (3a) de la lentille (3) en deux zones de contact opposées selon une deuxième direction diamétrale (16) distincte de la première direction diamétrale (15), cette deuxième paire de portées de pliage (10, 11, 11a, 11b) étant adaptée pour produire, sous l'effet de l'actionnement d'au moins un organe (18, 42, 51, 52, 55, 62a, 62b) mobile de manoeuvre selon le deuxième mode d'actionnement, un pliage de la lentille selon une deuxième ligne de pliage distincte de la première ligne de pliage,
de sorte que la lentille (3) peut être pliée selon l'une ou l'autre des différentes lignes de pliage.

2. Dispositif selon la revendication 1, **caractérisé en ce que** la première direction diamétrale (15) et la deuxième direction diamétrale (16) forment entre elles un angle compris entre 60° et 120°, de sorte que la lentille (3) peut être pliée selon l'une ou l'autre de deux lignes de pliage formant entre elles un angle compris entre 60° et 120°.

3. Dispositif selon l'une des revendications 1 et 2, **caractérisé en ce qu'**il comprend un bâti (1) comportant les moyens (2) de réception de la lentille (3), **en ce que** l'une au moins (9, 9a, 9b, 11, 11a, 11b) des deux portées de pliage, dite portée de pliage mobile (9, 9a, 9b, 11, 11a, 11b), d'au moins l'une des paires de portées de pliage est montée par rapport au bâti (1) de façon à pouvoir être rapprochée de l'autre portée de pliage (8, 9b, 9a, 10, 11b, 11a) de cette paire de portées de pliage, et **en ce que** les moyens (4) de manoeuvre comprennent au moins un organe (18, 42, 51, 52, 55, 62a, 62b) mobile de manoeuvre relié à au moins une portée de pliage (9, 9a, 9b, 11, 11a, 11b) mobile pour en commander les déplacements par rapport aux moyens (2) de réception lorsque cet organe mobile de manoeuvre est actionné.

4. Dispositif selon l'une des revendications 1 à 3, **caractérisé en ce que** les moyens (4) de manoeuvre comprennent au moins deux organes (51, 52) mobiles de manoeuvre, dont l'un (51) est relié à au moins une première portée de pliage mobile (9) d'une première paire de portées de pliage (8, 9), et dont l'autre (52) est relié à au moins une deuxième portée de pliage mobile (11) d'une deuxième paire de portées de pliage (10, 11).

5. Dispositif selon l'une des revendications 1 à 3, **caractérisé en ce que** les moyens (4) de manoeuvre comprennent un organe (18, 42, 55) mobile de manoeuvre relié à au moins une première portée de pliage mobile (9, 9a, 9b) de la première paire de portées de pliage (8, 9, 9a, 9b) et à au moins une deuxième portée de pliage mobile (11, 11a, 11b) de la deuxième paire de portées de pliage (10, 11, 11a, 11b), et adapté pour pouvoir être actionné soit selon le premier mode d'actionnement, soit selon le deuxième mode d'actionnement.

6. Dispositif selon la revendication 5, **caractérisé en ce que** l'organe (18) mobile de manoeuvre est monté rotatif par rapport au bâti (1), **en ce que** le premier mode d'actionnement correspond à un déplacement en rotation de l'organe (18) mobile de manoeuvre dans un premier sens de rotation (24), et **en ce que** le deuxième mode d'actionnement correspond à un déplacement en rotation de l'organe (18) mobile de manoeuvre dans le deuxième sens de rotation (25) opposé au premier sens de rotation (24).

7. Dispositif selon la revendication 5, **caractérisé en ce que** l'organe (42) mobile de manoeuvre est monté par rapport au bâti (1) de façon à pouvoir être déplacé selon au moins deux directions (49, 50) de translation distinctes dont l'une (49) correspond au premier mode d'actionnement alors que l'autre (50) correspond au deuxième mode d'actionnement.

8. Dispositif selon la revendication 5, **caractérisé en ce que** l'organe (55) mobile de manoeuvre est monté par rapport au bâti (1) de façon à pouvoir être déplacé selon au moins une direction de translation et selon au moins deux sens opposés (57, 58) selon cette direction de translation, dont l'un (57) correspond au premier mode d'actionnement alors que l'autre (58) correspond au deuxième mode d'actionnement.

9. Dispositif selon l'une des revendications 5 à 8, **caractérisé en ce qu'**une première portée de pliage mobile (9) de la première paire de portées de pliage (8, 9) et une deuxième portée de pliage mobile (11) de la deuxième paire de portées de pliage (10, 11) sont solidaires d'une seule et même pièce mobile intermédiaire (12) montée et guidée par rapport au bâti (1) et reliée à l'organe (18) mobile de manoeuvre de façon à pouvoir être entraînée en déplacement sous l'effet de l'actionnement de cet organe (18) mobile de manoeuvre selon l'un ou l'autre des modes d'actionnement.

10. Dispositif selon l'une des revendications 1 à 9, **caractérisé en ce qu'**un organe (18, 42, 55) mobile de manoeuvre est relié à chaque portée de pliage mobile (9, 9a, 9b, 11, 11a, 11b) dont il commande les déplacements par l'intermédiaire d'un système à came (22, 47, 48, 56) et coulisseau (23, 38, 39) de contact.

11. Dispositif selon l'une des revendications 1 à 10, **caractérisé en ce que** chaque paire de portées de pliage comprend une portée de pliage fixe (8, 10) par rapport au bâti (1).

12. Dispositif selon l'une des revendications 1 à 11, **caractérisé en ce que** chaque portée de pliage mobile (9, 9a, 9b, 11, 11a, 11b) est montée par rapport au bâti (1) de façon à pouvoir être mobile au moins sensiblement en translation.

13. Dispositif selon l'une des revendications 1 à 12, **caractérisé en ce que** les moyens (2) de réception sont adaptés pour recevoir la lentille (3) intraoculaire orientée selon une et une seule direction possible.

14. Dispositif selon l'une des revendications 1 à 13, **caractérisé en ce qu'**il constitue un emballage d'une lentille (3) intraoculaire et comprend un capot (30) refermé sur la lentille (3) en place dans les moyens (2) de réception, ce capot (30) étant adapté pour pouvoir être ouvert pour permettre l'accès à la lentille (3).

15. Dispositif selon la revendication 14, **caractérisé en ce que** le capot (30) est adapté pour, en position refermée sur la lentille (3), interdire tout déplacement intempestif des moyens (4) de manoeuvre, et, en position ouverte, autoriser l'actionnement des moyens (4) de manoeuvre selon l'un ou l'autre des modes d'actionnement.

## Patentansprüche

1. Vorrichtung zum Falten einer Intraokularlinse (3) mit flexiblem optischem Teil (3a) vor ihrer Implantation, die Folgendes umfasst:
. Mittel (2) zum Aufnehmen der Linse (3),
. wenigstens ein Paar Faltanschläge (8, 9, 9a, 9b, 10, 11, 11a, 11b), die so gestaltet sind, dass sie in Kontakt mit dem optischen Teil (3a) der Linse (3) in zwei gegenüberliegenden Kontaktzonen in derselben diametralen Richtung platziert werden können,
. Betätigungsmittel (4) in Verbindung mit wenigstens einem Faltanschlag (9, 9a, 9b, 11, 11a, 11b), die so gestaltet sind, dass sie bei Betätigung eine Bewegung in Richtung auf einen anderen Faltanschlag (8, 9b, 11b, 11a) desselben Paares von Faltanschlägen bewirken,
**dadurch gekennzeichnet, dass** die Betätigungsmittel (4) so gestaltet sind, dass, wenn sich eine Linse (3) in den Aufnahmemitteln (2) befindet:
- in einem ersten Arbeitsmodus der Betätigungsmittel (4) ein erstes Paar Faltanschläge (8, 9, 9a, 9b) in Kontakt mit der Peripherie des optischen Teils (3a) der Linse (3) in zwei gegenüberliegenden Kontaktzonen in einer ersten diametralen Richtung (15) kommt, wobei dieses erste Paar Faltanschläge (8, 9, 9a, 9b) so gestaltet ist, dass es unter der Wirkung der Arbeit von wenigstens einem beweglichen Betätigungsmechanismus (18, 42, 51, 52, 55, 62a, 62b) in diesem ersten Arbeitsmodus eine Faltung der Linse gemäß einer ersten Faltlinie erzeugt,
- in wenigstens einem zweiten Arbeitsmodus der Betätigungsmittel (4) ein zweites Paar Faltanschläge (10, 11, 11a, 11b), das sich von dem ersten Paar Faltanschläge unterscheidet, in Kontakt mit der Peripherie des optischen Teils (3a) der Linse (3) in zwei gegenüberliegenden Kontaktzonen in einer zweiten diametralen Richtung (16) kommt, die sich von der ersten diametralen Richtung (15) unterscheidet, wobei dieses zweite Paar Faltanschläge (10, 11, 11a, 11b) so gestaltet ist, dass unter der Wirkung der Arbeit von wenigstens einem beweglichen Betätigungsmechanismus (18, 42, 51, 52, 55, 62a, 62b) in dem zweiten Arbeitsmodus eine Faltung der Linse in einer zweiten Faltlinie erzeugt wird, die sich von der ersten Faltlinie unterscheidet,
so dass die Linse (3) in der einen oder der anderen der unterschiedlichen Faltlinien gefaltet werden kann.

2. Vorrichtung nach Anspruch 1, **dadurch gekennzeichnet, dass** die erste diametrale Richtung (15) und die zweite diametrale Richtung (16) zwischen sich einen Winkel zwischen 60° und 120° bilden, so dass die Linse (3) gemäß der einen oder anderen der beiden Faltlinien gefaltet werden kann, die zwischen sich einen Winkel zwischen 60° und 120° bilden.

3. Vorrichtung nach einem der Ansprüche 1 und 2, **dadurch gekennzeichnet, dass** sie ein Gehäuse (1) umfasst, umfassend die Mittel (2) zum Aufnehmen der Linse (3), dadurch, dass wenigstens einer (9, 9a, 9b, 11, 11a, 11b) der beiden Faltanschläge, beweglicher Faltanschlag (9, 9a, 9b, 11, 11a, 11b) genannt, von wenigstens einem der Paare von Faltanschlägen in Bezug auf das Gehäuse (1) so montiert ist, dass er sich dem anderen Faltanschlag (8, 9b, 9a, 10, 11b, 11a) dieses Paares von Faltanschlägen nähern kann, und dadurch, dass die Betätigungsmittel (4) wenigstens einen beweglichen Betätigungsmechanismus (18, 42, 51, 52, 55, 62a, 62b) umfassen, der mit wenigstens einem beweglichen Faltanschlag (9, 9a, 9b, 11, 11a, 11b) verbunden ist, um die Bewegungen in Bezug auf die Aufnahmemittel (2) zu bewirken, wenn dieser bewegliche Betätigungsmechanismus betätigt wird.

4. Vorrichtung nach einem der Ansprüche 1 bis 3, **dadurch gekennzeichnet, dass** die Betätigungsmittel (4) wenigstens zwei bewegliche Betätigungsmechanismen (51, 52) umfassen, von denen einer (51) mit wenigstens einem ersten beweglichen Faltanschlag (9) eines ersten Paares von Faltanschlägen (8, 9) und der andere (52) mit wenigstens einem zweiten beweglichen Faltanschlag (11) eines zweiten Paares von Faltanschlägen (10, 11) verbunden ist.

5. Vorrichtung nach einem der Ansprüche 1 bis 3, **dadurch gekennzeichnet, dass** die Betätigungsmittel (4) einen beweglichen Betätigungsmechanismus (18, 42, 55) umfassen, der mit wenigstens einem ersten beweglichen Faltanschlag (9, 9a, 9b) des ersten Paares von Faltanschlägen (8, 9, 9a, 9b) und mit wenigstens einem zweiten beweglichen Faltanschlag (11, 11a, 11b) des zweiten Paares von Faltanschlägen (10, 11, 11a, 11b) verbunden und so gestaltet ist, dass er entweder im ersten Arbeitsmodus oder im zweiten Arbeitsmodus betätigt werden kann.

6. Vorrichtung nach Anspruch 5, **dadurch gekennzeichnet, dass** der bewegliche Betätigungsmechanismus (18) drehbar in Bezug auf das Gehäuse (1) montiert ist, dadurch, dass der erste Arbeitsmodus einer Rotationsbewegung des beweglichen Betätigungsmechanismus (18) in einer ersten Drehrichtung (24) entspricht, und dadurch, dass der zweite Arbeitsmodus einer Drehbewegung des beweglichen Betätigungsmechanismus (18) in der zweiten Drehrichtung (25) entspricht, die der ersten Drehrichtung (24) entgegengesetzt ist.

7. Vorrichtung nach Anspruch 5, **dadurch gekennzeichnet, dass** der bewegliche Betätigungsmechanismus (42) in Bezug auf das Gehäuse (1) so montiert ist, dass er in wenigstens zwei verschiedenen Translationsrichtungen (49, 50) bewegt werden kann, von denen die eine (49) einem ersten Arbeitsmodus entspricht, während die andere (50) einem zweiten Arbeitsmodus entspricht.

8. Vorrichtung nach Anspruch 5, **dadurch gekennzeichnet, dass** der bewegliche Betätigungsmechanismus (55) in Bezug auf das Gehäuse (1) so montiert ist, dass er in wenigstens einer Translationsrichtung und wenigstens zwei dieser Translationsrichtung entgegengesetzten Richtungen (57, 58) bewegt werden kann, von denen die eine (57) dem ersten Arbeitsmodus entspricht, während die andere (58) dem zweiten Arbeitsmodus entspricht.

9. Vorrichtung nach einem der Ansprüche 5 bis 8, **dadurch gekennzeichnet, dass** ein erster beweglicher Faltanschlag (9) des ersten Paares von Faltanschlägen (8, 9) und ein zweiter beweglicher Faltanschlag (11) des zweiten Paares von Faltanschlägen (10, 11) aus einem Stück mit einem beweglichen Zwischenstück (12) ausgebildet sind, das in Bezug auf das Gehäuse (1) montiert ist und geführt wird und mit dem beweglichen Betätigungsmechanismus (18) so verbunden ist, dass es unter der Wirkung der Arbeit dieses beweglichen Betätigungsmechanismus (18) in dem einen oder dem anderen Arbeitsmodus bewegt werden kann.

10. Vorrichtung nach einem der Ansprüche 1 bis 9, **dadurch gekennzeichnet, dass** ein beweglicher Betätigungsmechanismus (18, 42, 55) mit jedem beweglichen Faltanschlag (9, 9a, 9b, 11, 11a, 11b) verbunden ist, dessen Bewegungen er mit einem System aus Nocken (22, 47, 48, 56) und Kontaktschieber (23, 38, 39) bewirkt.

11. Vorrichtung nach einem der Ansprüche 1 bis 10, **dadurch gekennzeichnet, dass** jedes Paar Faltanschläge einen festen Faltanschlag (8, 10) in Bezug auf das Gehäuse (1) umfasst.

12. Vorrichtung nach einem der Ansprüche 1 bis 11, **dadurch gekennzeichnet, dass** jeder bewegliche Faltanschlag (9, 9a, 9b, 11, 11a, 11b) in Bezug auf das Gehäuse (1) so montiert ist, dass er wenigstens im Wesentlichen translationsbeweglich ist.

13. Vorrichtung nach einem der Ansprüche 1 bis 12, **dadurch gekennzeichnet, dass** die Aufnahmemittel (2) so gestaltet sind, dass sie die Intraokularlinse (3) in nur einer möglichen Orientierungsrichtung aufnehmen können.

14. Vorrichtung nach einem der Ansprüche 1 bis 13, **dadurch gekennzeichnet, dass** sie eine Umhüllung für eine Intraokularlinse (3) darstellt und eine Kappe (30) aufweist, die auf der in den Aufnahmemitteln (2) befindlichen Linse (3) geschlossen wird, wobei diese Kappe (30) so gestaltet ist, dass sie geöffnet werden kann, damit die Linse (3) zugängig ist.

15. Vorrichtung nach Anspruch 14, **dadurch gekennzeichnet, dass** die Kappe (30) so gestaltet ist, dass in der geschlossenen Position auf der Linse (3) jede ungewollte Bewegung der Betätigungsmittel (4) verhütet und in der offenen Position die Betätigung der Betätigungsmittel (4) in dem einen oder dem anderen Arbeitsmodus zugelassen wird.

## Claims

1. A device for bending an intraocular lens (3) with a flexible optical part (3a), before the lens is implanted, comprising:
means (2) for reception of the lens (3);
at least one pair of bending support surfaces (8,9,9a,9b,10,11,11a,11b), which are designed to be able to be placed in contact with the optical part (3a) of the lens (3), in two areas of contact which are opposite according to a single diametral direction;
means (4) for manoeuvring, which are connected to at least one bending support surface (9,9a,9b,11,11a,11b), and, when they are actuated, are designed to control displacement towards another bending support surface (8,9b,11b,11a) of a single pair of bending support surfaces,
**characterized in that** the means (4) for manoeuvring are designed such that, with a lens (3) in place in the means (2) for reception:
- according to a first method for actuation of the means (4) for manoeuvring, a first pair of bending support surfaces (8,9,9a,9b) comes into contact with the periphery of the optical part (3a) of the lens (3), in two areas of contact which are opposite according to a first diametral direction (15), this first pair of bending support surfaces (8,9,9a,9b) being designed, under the effect of actuation of at least one mobile manoeuvring unit (18,42,51,52,55,62a,62b) according to this first method for actuation, to produce bending of the lens according to a first bending line;
- according to at least a second method for actuation of the means (4) for manoeuvring, a second pair of bending support surfaces (10,11,11a,11b), which is distinct from the first pair of bending support surfaces, comes into contact with the periphery of the optical part (3a) of the lens (3), in two areas of contact which are opposite according to a second diametral direction (16), which is different from the first diametral direction (15), this second pair of bending support surfaces (10,11,11a,11b) being designed, under the effect of actuation of at least one mobile manoeuvring unit (18,42,51,52,55,62a,62b) according to the second method for actuation, to produce bending of the lens according to a second bending line, which is distinct from the first bending line,
such that the lens (3) can be bent according to one or the other of the different bending lines.

2. A device as claimed in claim 1, **characterized in that** the first diametral direction (15) and the second diametral direction (16) form between one another an angle of between 60° and 120°, such that the lens (3) can be bent according to one or the other of two bending lines which form between one another an angle of between 60° and 120°.

3. A device as claimed in one of claims 1 and 2,
**characterized in that** it comprises a frame (1) which includes the means (2) for reception of the lens (3), **in that** at least one (9,9a,9b,11,11a,11b) of the two bending support surfaces, known as the mobile bending support surface (9,9a,9b,11,11a,11b) of at least one of the pairs of bending support surfaces, is fitted relative to the frame (1) such that it can be drawn closer to the other bending support surface (8,9b,9a,10,11b,11a) of this pair of bending support surfaces, and **in that** the means (4) for manoeuvring comprise at least one mobile manoeuvring unit (18,42,51,52,55,62a,62b), which is connected to at least one mobile bending support surface (9,9a,9b,11,11a,11b), in order to control the displacements of the latter relative to the means (2) for reception, when this mobile manoeuvring unit is actuated.

4. A device as claimed in any one of claims 1 to 3,
**characterized in that** the means (4) for manoeuvring comprise at least two mobile manoeuvring units (51,52), one of which (51) is connected to at least one first mobile bending support surface (9) of a first pair of bending support surfaces (8,9), and the other (52) of which is connected to at least one second mobile bending support surface (11) of a second pair of bending support surfaces (10,11).

5. A device as claimed in any one of claims 1 to 3,
**characterized in that** the means (4) for manoeuvring comprise a mobile manoeuvring unit (18,42,55), which is connected to at least one first mobile bending support surface (9,9a,9b) of the first pair of bending support surfaces (8,9,9a,9b), and to at least one second mobile bending support surface (11,11a,11b) of the second pair of bending support surfaces (10,11,11a,11b), and is designed to be able to be actuated either according to the first method for actuation, or according to the second method for actuation.

6. A device as claimed in claim 5, **characterized in that** the mobile manoeuvring unit (18) is fitted such as to rotate relative to the frame (1), **in that** the first method for actuation corresponds to displacement in rotation of the mobile manoeuvring unit (18) in a first direction of rotation (24), and **in that** the second method for actuation corresponds to displacement in rotation of the mobile manoeuvring unit (18) in the second direction of rotation (25), opposite to the first direction of rotation (24).

7. A device as claimed in claim 5, **characterized in that** the mobile manoeuvring unit (42) is fitted relative to the frame (1) such that it can be displaced according to at least two distinct directions (49,50) of translation, one of which (49) corresponds to the first method for actuation, whereas the other (50) corresponds to the second method for actuation.

8. A device as claimed in claim 5, **characterized in that** the mobile manoeuvring unit (55) is fitted relative to the frame (1) such that it can be displaced according to at least one direction of translation, and according to at least two opposite directions (57,58) according to this direction of translation, one of which (57) corresponds to the first method for actuation, whereas the other (58) corresponds to the second method for actuation.

9. A device as claimed in any one of claims 5 to 8,
**characterized in that** a first mobile bending support surface (9) of the first pair of bending support surfaces (8,9) and a second mobile bending support surface (11) of the second pair of bending support surfaces (10,11) are integral with a single intermediate mobile part (12), which is fitted and guided relative to the frame (1), and is connected to the mobile manoeuvring unit (18), such as to be able to be entrained in displacement under the effect of actuation of this mobile manoeuvring unit (18) according to one or the other of the methods for actuation.

10. A device as claimed in any one of claims 1 to 9,
**characterized in that** a mobile manoeuvring unit (18,42,55) is connected to each mobile bending support surface (9,9a,9b,11,11a,11b) of which it controls the displacements, by means of a cam (22,47,48,56) and contact slide system (23,38,39).

11. A device as claimed in any one of claims 1 to 10,
**characterized in that** each pair of bending support surfaces comprises a bending support surface (8,10) which is fixed relative to the frame (1).

12. A device as claimed in any one of claims 1 to 11,
**characterized in that** each mobile bending support surface (9,9a,9b,11,11a,11b) is fitted relative to the frame (1), such as to be able to be mobile at least substantially in translation.

13. A device as claimed in any one of claims 1 to 12,
**characterized in that** the means (2) for reception are designed to receive the intraocular lens (3) which is oriented according to a single possible direction only.

14. A device as claimed in any one of claims 1 to 13,
**characterized in that** it constitutes packaging for an intraocular lens (3), and includes a cap (30) which is closed onto the lens (3) which is in place in the means (2) for reception, this cap (30) being designed to be able to be opened in order to permit access to the lens (3).

15. A device as claimed in claim 14, **characterized in that** the cap (30) is designed such that, in the position in which it is closed onto the lens (3), it prevents any premature displacement of the means (4) for manoeuvring, and, in its open position, it permits actuation of the means (4) for manoeuvring according to one or the other of the methods for actuation.
